# EUROPÄISCHE PATENTSCHRIFT

(11) **EP 1 019 102 B1**
(45) Veröffentlichungstag und Bekanntmachung des Hinweises auf die Patenterteilung: **02.05.2002**
(21) Anmeldenummer: 98952659.5
(22) Anmeldetag: 29.09.1998
(51) Int. Cl.: A61L 15/00, A61P 7/02

(54) **32 P- POLYPHOSPHAZEN - RADIOAKTIV MARKIERTES ANTITHROMBOGENES POLYMER**
32 P - POLYPHOSPHAZENE - RADIOLABELLED ANTITHROMBOGENIC POLYMER
32 P - POLYPHOSPHAZENE - POLYMERE ANTITHROMBOGENIQUE RADIOMARQUE

(30) Priorität: 30.09.1997 DE 19743373
(43) Veröffentlichungstag der Anmeldung: 19.07.2000
(73) Patentinhaber: Universität Heidelberg, 69117 Heidelberg (DE)
(72) Erfinder: GRUNZE, Michael, D-69151 Neckargemünd (DE); WELLE, Alexander, D-68525 Ladenburg (DE)
(74) Vertreter: Müller-Boré & Partner Patentanwälte
(86) Internationale Anmeldenummer: EP9806167
(87) Internationale Veröffentlichungsnummer: WO9916477

(56) Entgegenhaltungen:
- WO-A-95/28966
- WO-A-96/04015
- WO-A-96/29059
- DE-A- 19 613 048
- US-A- 4 880 622

## Beschreibung

Die vorliegende Erfindung betrifft ein radioaktiv markiertes, antithrombogenes Polymer und dessen Verwendung als Bestandeil therapeutischer Vorrichtungen zur Verhinderung von übermäßiger Zellproliferation oder Narbenbildung sowie Vorrichtungen, welche das radioaktiv markierte, antithrombogene Polymer umfassen, wie bespielsweise Pflaster oder künstliche Implantate mit einem biokompatiblen Überzug.

Die größten Komplikationen, die durch künstliche Implantate auftreten, sind zum einen in der vermehrten Thrombozytenablagerung auf der körperfremden Oberfläche zu sehen, zum anderen in der verstärkten Zellproliferation (Narbenbildung) am verletzten und heilenden Gewebe, mit dem das künstliche Implantat verbunden ist.

Die Thrombenbildung beim Kontakt von menschlichem Blut mit der körperfremden Oberfläche, beispielsweise künstliche Herzklappen, ist im Stand der Technik beschrieben (vgl. Informationsmaterial der Firma Metronic Hall, Bad Homburg, Carmeda BioAktive Oberfläche (CBSA), Seite 1-21 und Buddy D. Ratner, "The blood compatibility catastrophe", Journal of Biomedical Materials Research, Vol. 27., 283-287 sowie Cary W., Akins, MD, "Mechanical Cardiac Valvular Prostheses", The Society of Thoracic Surgeons, 161-171, 1991). Beispielsweise bestehen die auf dem Weltmarkt befindlichen künstlichen Herzklappen aus pyrolisiertem Kohlenstoff und zeigen eine erhöhte Neigung zur Ausbildung von Thromben (vgl. Cary W., Akins, s.o.). Bei blutkontaktierenden Implantaten, z.B. künstlichen Herzklappen, treten zur Zeit neben Thrombosen auch schwerwiegende medizinische Probleme durch Embolien und Entzündungen (Endocarditis) auf.

Bei Gefäßimplantaten, z.B. sogenannten Stents, treten neben den bereits bekannten Problemen der verstärkten Thrombenbildung Restenosen (d.h. die Wiederverengung des Blutgefäßes im durch eine Angioplastie aufgedehnten Bereich, häufig der Stentbereich) auf. Diese Komplikationen werden infolge der Aktivierung des Gerinnungs- und Immunsystems durch den implantierten Fremdkörper sowie eine Schädigung der Gefäßwand bei der Stentimplantation im Verlauf einer Angioplastie ausgelöst. Gegenwärtig erhalten daher Patienten mit künstlichen Herzklappen, wie auch während der postoperativen Behandlung nach einer Angioplastie, Gerinnungshemmer (Vitamin K Antagonisten), deren Dosierung jedoch problematisch ist. Die Verwendung von Stents ist gegenwärtig aufgrund von Thrombenbildung in engen (d<4mm) und venösen Blutgefäßen unmöglich. Bei Implantation in Arterien tritt durch starke Gewebeproliferation (Intimahyperplastie) oft eine erneute Gefäßverengung im Stentbereich (Restenose) auf. Die Restenosehäufigkeit beträgt bei handelsüblichen Stents ca. 30-50% innerhalb von 6 Monaten nach erfolgter Angioplastie. Hehrlein et al. konnten zeigen, daß die Restenosehäufigkeit durch die Anwendung radioaktiver Strahlung erheblich verringert wird. In ihren Versuchen setzen Hehrlein et al. als radioaktive Quelle ³²P Ionen ein, die mittels lonenimplantation in das metallische Stentmaterial (Ti/Ni Legierungen, Tantal, Chirurgischer Stahl) eingebracht wurden. Die so emittierte β-Strahlung besitzt im Gewebe nur geringe Reichweiten (einige mm), wird aber, im Gegensatz zu γ-Strahlung, vom Gewebe sehr gut absorbiert und ist deshalb sehr effektiv. Diese Eigenschaft der β-Strahlung ermöglicht es, die Gesamtstrahlenbelastung des Patienten sehr gering zu halten (applizierte Aktivität < 10 µCi, erlaubte jährliche orale Aufnahme von ³²P: 600 µCi, bei Stents übertragene integrale Strahlendosis etwa 700 gray) und die Strahlung, somit auch den Therapiebereich, lokal zu begrenzen. Weiterhin sind die für den behandelnden Arzt sowie für den Transport, etc, erforderlichen Schutzmaßnahmen vergleichsweise gering. Jedoch ist die Methode der Ionenimplantation für Stents technisch aufwendig und kostenintensiv, außerdem löst diese Behandlung alleine das Problem der Thrombenbildung am Implantat nicht.

Die polymere Verbindung Poly[bis(trifluoroethoxy)phosphazen] zeigt als Volumenmaterial eine gute antithrombogene Wirkung (vgl. Tur, Untersuchungen zur Thrombenresistenz von Poly[bis(trifluoroethoxy)phosphazen] und Hollemann Wiberg, "Stickstoffverbindungen des Phosphors" Lehrbuch der anorganischen Chemie, 666-669, 91.-100. Auflage, Walter de Gruyter Verlag, 1985, sowie Tur, Vinogradova, u.a. "Entwicklungstendenzen bei polymeranalogen Umsetzungen von Polyphosphazen", Acta Polymerica 39, 424-429, Nr. 8, 1988). Weiterhin wurden Polyphosphazene in der Patentschrift DE 196 13 048 als Beschichtung für die Beschichtung künstlicher Implantate eingesetzt, ohne daß die Möglichkeit, dieses Material durch entsprechende Veränderung therapeutisch wirksam zu gestalten, in Erwägung gezogen wurde. Auch kann diese Substanz alleine das Zellwachstum, welches zu Restenosen führt, nicht begrenzen oder verringern. Weiterhin weist diese polymere Verbindung als reines Volumenmaterial nicht die Härte und mechanische Belastbarkeit, die beispielsweise für künstliche Herzklappen oder Stents gefordert werden. In Kombination mit der therapeutischen Wirkung von radioaktiver Strahlung aber kann ihr Einsatz auch in anderen Implantaten oder therapeutischen Geräten bzw. Vorrichtungen, welche auf eine Verhinderung von übermäßiger Zellproliferation ausgerichtet sind, erfolgen.

Somit liegt der vorliegenden Erfindung die Aufgabe zugrunde, ein Material für medizinische Vorrichtungen, beispielsweise Katheter, Pflaster, Implantate etc., sowie deren Beschichtung bereitzustellen, welches einerseits ausgezeichnete mechanische Eigenschaften und antithrombogene Eigenschaften aufweisen soll, um dadurch die Biokompatibilität derartiger Vorrichtungen zu verbessern, andererseits sollen auch die vorgenannten Folgeschäden nach erfolgter Behandlung bzw. Implantation verhindert oder verringert werden. Insbesondere soll unkontrolliertes Zellwachstum, welches beispielsweise nach Stentimplantation zu Restenosen führt, verhindert bzw. verringert werden.

Diese Aufgabe wird durch die Bereitstellung eines antithrombogenen Polymers mit der folgenden allgemeinen Formel (I) gelöst, 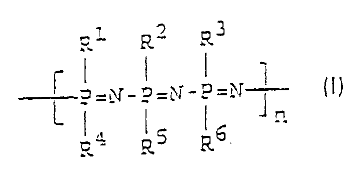 wobei n für 2 bis ∞ steht, R¹ bis R⁶ gleich oder unterschiedlich sind und einen Alkoxy-, Alkylsulfonyl-, Dialkylamino- oder Aryloxyrest oder einen Heterocycloalkyl- oder Heteroarylrest mit Stickstoff als Heteroatom bedeuten und wobei mindestens ein Teil der Polymerkette des antithrombogenen Polymers einen radioaktiv markierten Bestandteil enthält.

Vorzugsweise emittiert der im antithrombogenen Polymer enthaltene, radioaktiv markierte Bestandteil beim radioakiven Zerfall β-Strahlung. Jedoch kann je nach verwendetem Isotop auch γ-Strahlung emittiert werden. In einer bevorzugten Ausführungsform enthält das antithrombogene Polymer ein radioaktives Phosphorisotop. Noch mehr bevorzugt ist das antithrombogene Polymer ³²Pmarkiert. Das Phosphorisotop kann statistisch über das Polyphosphazenrückgrat verteilt sein. In einer anderen Ausführungsform ist jeder Phosphor im Polyphosphazenrückgrat, d.h. in der Polymerkette des antithrombogenen Polymers, ein radioaktives Phosphorisotop. In einer weiteren Ausführungsform kann der Phosphor in dem antithrombogenen Polymer teilweise durch ein radioaktives Aslsotop, bevorzugt ⁷⁶As, oder ein radioaktives Sb-lsotop, bevorzugt ¹²²Sb, ersetzt sein, wobei die Isotope statistisch über die Polymerkette des antithrombogenen Polymers verteilt sein können.

³²P ist ein β-Strahler mit einer maximalen Energie von 1,7 MeV, einer maximalen spezifischen Aktivität von 9000Ci/mmol und einer Halbwertszeit von 14,29 Tagen. Die maximale Reichweite der von ³²P emittierten β-Strahlung beträgt in Luft etwa 8 m. Das im Gewebe zu 80-90 % vorliegende Wasser reicht jedoch als als Abschirmung aus, um die emittierte Strahlung derart abzuschwächen, daß im Körpergewebe nur eine Eindringtiefe von maximal einigen Millimetern erreicht wird. Die vom Phosphorisotop emittierte β-Strahlung verringert unkontrolliertes Zellwachstum, welches beispielsweise nach einer Stentimplantation zu Restenosen führt. Dieser Effekt kann aber auch durch die Anwendung von γ-Strahlung, wie sie beispielsweise von ⁷⁶As oder ¹²²Sb emittiert wird, erreicht werden.

Wie zuvor angeführt kann der Polymerisationsgrad des erfindungsgemäßen Polymers 2 bis ∞ betragen. Bevorzugt ist jedoch ein Bereich für den Polymerisationsgrad von 20 bis 150000, mehr bevorzugt von 40 bis 70000.

Vorzugsweise ist mindestens einer der Reste R¹ bis R⁶ im antithrombogenen Polymer ein Alkoxyrest, der mit mindestens einem Fluoratom substituiert ist.

Die Alkylreste in den Alkoxy-, Alkylsulfonyl- und Dialkylaminoresten sind beispielsweise gerad- oder verzweigtkettige Alkylreste mit 1 bis 20 Kohlenstoffatomen, wobei die Alkylreste beispielsweise mit mindestens einem Halogenatom, wie ein Fluoratom, substituiert sein können.

Beispiele für Alkoxyreste sind Methoxy-, Ethoxy-, Propoxy- und Butoxygruppen, die vorzugsweise mit mindestens einem Fluoratom substituiert sein können. Besonders bevorzugt ist die 2,2,2-Trifluoroethoxygruppe. Beispiele für Alkylsulfonylreste sind Methyl-, Ethyl-, Propyl- und Butylsulfonylgruppen. Beispiele für Dialkylaminoreste sind Dimethyl-, Diethyl-, Dipropyl- und Dibutylaminogruppen.

Der Arylrest im Aryloxyrest ist beispielsweise eine Verbindung mit einem oder mehreren aromatischen Ringsystemen, wobei der Arylrest beispielsweise mit mindestens einem, vorstehend definierten Alkylrest substituiert sein kann. Beispiele für Aryloxyreste sind Phenoxy- und Naphthoxygruppen und Derivate davon.

Der Heterocycloalkylrest ist beispielsweise ein 3- bis 7- Atome enthaltendes Ringsystem, wobei mindestens ein Ringatom ein Stickstoffatom ist. Der Heterocycloalkylrest kann beispielsweise mit mindestens einem, vorstehend definierten Alkylrest substituiert sein. Beispiele für Heterocycloalkylreste sind Piperidinyl-, Piperazinyl-, Pyrrolidinyl- und Morpholinylgruppen und Derivate davon. Der Heteroarylrest ist beispielsweise eine Verbindung mit einem oder mehreren aromatischen Ringsystemen, wobei mindestens ein Ringatom ein Stickstoffatom ist. Der Heteroarylrest kann beispielsweise mit mindestens einem, vorstehend definierten Alkylrest substituiert sein. Beispiele für Heteroarylreste sind Pyrrolyl-, Pyridinyl-, Pyridinolyl-, Isochinolinyl- und Chinolinylgruppen, und Derivate davon.

In einer bevorzugten Ausführungsform der vorliegenden Erfindung ist das antithrombogene Polymer ein mit ³²P- oder As- oder Sb - Isotopen markiertes Poly[bis(trifluoroethoxy)phosphazen].

Ein weiterer Gegenstand der vorliegenden Erfindung ist ein erfindungsgemäßes antithrombogenes Polymer mit der allgemeinen Formel (I) zur Verwendung als Bestandteil therapeutischer Vorrichtungen zur Verhinderung von übermäßiger Zellproliferation oder Narbenbildung oder zur Tumorbehandlung. Insbesondere kann das erfindungsgemäße antithrombogene Polymer mit der allgemeinen Formel (I) als Bestandteil therapeutischer Vorrichtungen, wie künstliche Implantante, Pflaster, Herzklappen, künstlichen Blutgefäße, Stents, Katheter, Urether oder sonstigen Implantate ohne direkten Blutkontakt, verwendet werden.

Das erfindungsgemäße antithrombogene Polymers kann jedoch nicht nur als Beschichtung, sondern in besonderen Anwendungsfällen, beispielsweise in der Anwendung für endovaskuläre Prothesen o.ä., auch als Vollmaterial verwendet werden. Weiterhin kann dieses Material nicht nur zur Anwendung in arteriellen Gefäßen, songern auch in der Anwendung in venösen Gefäßen sowie ganz allgemein für die Beschichtung von Implantaten jedweden Typs verwendet werden.

Ferner wird gemäß der vorliegenden Erfindung eine therapeutische Vorrichtung bereitgestellt, welches das erfindungsgemäße antithrombogene Polymer umfasst. Beispielsweise sind solche therapeutische Vorrichtungen ein Pflaster bzw. Pflasterzusatz, welches bzw. welcher insbesondere zur Therapie verstärkter Zellproliferation während der Wundheilung (sogenannte Keloide) oder zur Behandlung von verschiedenen Formen von Hautkrebs therapeutische Anwendung finden kann, oder ein künstliches Implantat.

In einer bevorzugten Ausführungsform der vorliegenden Erfindung wird ein künstliches Implantatmaterial bereitgestellt, das ein Implantatmaterial als Substrat und einen mindestens teilweise auf die Oberfläche des Substrats aufgebrachten, biokompatiblen Überzug umfaßt, der das radioaktiv markierte, antithrombogene Polymer mit der vorgenannten allgemeinen Formel (I) enthält.

Der biokompatible Überzug des erfindungsgemäßen künstlichen Implantats weist beispielsweise eine Dicke von etwa 1 nm bis etwa 100 µm, vorzugsweise bis etwa 10 µm und besonders bevorzugt bis etwa 1 µm auf.

Das als Substrat erfindungsgemäß verwendete Implantatmaterial weist keine besondere Beschränkung auf und kann jedes Implantatmaterial, wie Kunststoffe, Metalle, Metallegierungen und Keramiken, sein. Beispielsweise kann das Impiantatmaterial eine künstliche Herzklappe aus pyrolysiertem Kohlenstoff oder ein metallisches Stentmaterial sein.

In einer Ausführungsform des erfindungsgemäßen künstlichen Implantats ist zwischen der Oberfläche des Substrats und dem das radioaktiv markierte Polyphosphazenderivat enthaltenden, biokompatiblen Überzug eine Schicht angeordnet, die einen Adhäsionspromotor enthält.

Der Adhäsionspromotor bzw. Spacer ist beispielsweise eine Silicium-organische Verbindung, vorzugsweise ein aminoterminiertes Silan bzw. basierend auf Aminosilan, oder eine Alkylphosphonsäure. Besonders bevorzugt ist Aminopropyltrimethoxysilan.

Der Adhäsionspromotor verbessert insbesondere die Haftung des Überzugs auf der Oberfläche des Vorrichtungs- bzw. Implantatmaterials durch Kopplung des Adhäsionspromotors an die Oberfläche des Implantatmaterials, beispielsweise über ionische und/oder kovalente Bindungen und durch weitere Kopplung des Adhäsionspromotors an reaktive Bestandteile, insbesondere an das radioaktiv markierte, antithrombogene Polymer des Überzugs, beispielsweise über ionische und/oder kovalente Bindungen.

Zur Herstellung des erfindungsgemäßen künstlichen Implantats wird radioaktiv markiertes Polydichlorphosphazen auf die Oberfläche des Substrats aufgebracht wird und mit mindestens einer reaktiven Verbindung, ausgewählt aus aliphatischen oder aromatischen Alkoholen oder deren Salze, Alkylsulfonen, Dialkylaminen und aliphatischen oder aromatischen Heterocyclen mit Stickstoff als Heteroatom, umgesetzt.

Die aliphatischen Alkohole sind beispielsweise gerad- oder verzweigtkettige, einoder mehrwertige Alkohole mit 1 bis 20 Kohlenstoffatomen, wobei die Alkohole beispielsweise mit mindestens einem Halogenatom, wie ein Fluoratom, substituiert sein können. Als deren Salze können beispielsweise Alkoholate mit Alkalimetallen als Kation verwendet werden. Vorzugsweise wird das aufgebrachte radioaktiv markierte Polydichlorphosphazen mit 2,2,2-Natriumtrifluorethanolat als reaktive Verbindung verestert.

Die Alkylreste der Alkylsulfone und Dialkylamine sind beispielsweise gerad- oder verzweigtkettige Alkylreste mit 1 bis 20 Kohlenstoffatomen, wobei die Alkylreste beispielsweise mit mindestens einem Halogenatom, wie ein Fluoratom, substituiert sein können.

Beispiele für Alkylsulfone sind Methyl-, Ethyl-, Propyl- und Butylsulfone. Beispiele für Dialkylamine sind Dimethyl-, Diethyl-, Dipropyl- und Dibutylamine. Die aromatischen Alkohole sind beispielsweise Verbindungen mit einem oder mehreren aromatischen Ringsystemen, wobei die aromatischen Alkohole beispielsweise mit mindestens einem, vorstehend definierten Alkylrest substituiert sein können. Beispiele für aromatische Alkohole bzw. deren Salze sind Phenol oder Phenolate und Naphthole oder Naphtholate.

Die aliphatischen Heterocyclen sind beispielsweise 3- bis 7-Atome enthaltende Ringsysteme, wobei mindestens ein Ringatom ein Stickstoffatom ist. Die aliphatischen Heterocyclen können beispielsweise mit mindestens einem, vorstehend definierten Alkylrest substituiert sein. Beispiele für aliphatische Heterocyclen sind Piperidin, Piperazin, Pyrrolidin und Morpholin, und Derivate davon.

Die aromatischen Heterocyclen sind beispielsweise Verbindungen mit einem oder mehreren aromatischen Ringsystemen, wobei mindestens ein Ringatom ein Stickstoffatom ist. Die aromatischen Heterocyclen können beispielsweise mit mindestens einem, vorstehend definierten Alkylrest substituiert sein. Beispiele für aromatische Heterocyclen sind Pyrrol, Pyridin, Pyridinol, Isochinolin und Chinolin, und Derivate davon.

Die Herstellung von Poly[bis(trifluoroethoxy)phosphazen], ausgehend von Hexachlorcyclotriphosphazen, ist im Stand der Technik bekannt. Die Polymerisation von Hexachlorcyclotriphosphazen wird ausführlich in Korsak, Vinogradova, Tur, Kasarova, Komarova und Gilman, "Über den Einfluß von Wasser auf die Polymerisation von Hexachlorcyclotriphosphazen", Acta Polymerica 30, Heft 5, Seite 245-248, 1979, beschrieben. Die Veresterung des durch die Polymerisation hergestellten Polydichlorphosphazen wird in Fear, Thower, Veitch in Journal of Chemical Society, Seite 1324, 1958, beschrieben.

Die erfindungsgemäß verwendeten, radioaktiv markierten Polyphosphazenderivate lassen sich durch Kondensation von ³²P-markiertem Phosphorpentachlorid, entweder als Reinsubstanz oder im Gemisch mit nicht-markiertem Phosphorpentachlorid, mit Ammoniumchlorid aufbauen. Weiterhin können in diesem Schritt Radioisotope des Arsenpentachlorids oder Antimonpentachlorids eingebracht werden. Die Menge an Radioisotop, einige µg dieser Isotope, richtet sich nach der gewünschten Aktivität und beeinflußt die mechanischen, chemischen und antithrombotischen Eigenschaften des Polyphosphazenderivats nicht.

Im nächsten Schritt schließt sich daran die Polymerisation des im vorstehenden Schritt erhaltenen, radioaktiv markierten Hexachlorcyclotriphosphazens gemäß den im vorgenannten Stand der Technik beschriebenen Methoden an. Die Veresterung des durch die Polymerisation hergestellten, radioaktiv markierten Polydichlorphosphazens erfolgt anschließend analog den im vorgenannten Stand der Technik beschriebenen Methoden.

Zur Herstellung der erfindungsgemäßen künstlichen Implantate wird ein vorstehend definierter Adhäsionspromotor auf die Oberfläche des Substrats aufgebracht und beispielsweise über ionische und/oder kovalente Bindungen an die Oberfläche gekoppelt. Danach wird das radioaktiv markierte Polydichlorphosphazen auf die mit dem Adhäsionspromotor beschichtete Oberfläche des Substrats aufgebracht und beispielsweise über ionische und/oder kovalente Bindungen an das radioaktiv markierte Polydichlorphosphazen gekoppelt. Anschließend wird das radioaktiv markierte Polydichlorphosphazen mit mindestens einer vorstehend definierten reaktiven Verbindung umgesetzt.

Vorzugsweise wird zur Herstellung der erfindungsgemäßen künstlichen Implantate radioaktiv markiertes Polydichlorphosphazen unter Inertgasatmosphäre auf die Oberfläche des Substrats aufgebracht, gegebenenfalls an den Adhäsionspromotor gekoppelt und mit der reaktiven Verbindung umgesetzt. Ferner kann das radioaktiv markierte Polydichlorphosphazen unter verringertem Druck oder unter Luftatmosphäre aufgebracht und gegebenenfalls an den Adhäsionspromotor gekoppelt werden.

Zur Herstellung der erfindungsgemäßen künstlichen Implantate kann das radioaktiv markierte Polydichlorphosphazen naßchemisch bzw. in Lösung oder aus der Schmelze oder durch Sublimation oder durch Aufsprühen aufgebracht und gegebenenfalls an den Adhäsionspromotor gekoppelt werden.

Der Adhäsionspromotor kann naßchemisch bzw. in Lösung oder aus der Schmelze oder durch Sublimation oder durch Aufsprühen auf das Substrat aufgebracht werden. Die naßchemische Ankopplung eines Adhäsionspromotors, vorzugsweise basierend auf Aminosilanen an hydroxylierte Oberflächen, ist in Marco Mantar, Diplomarbeit, S. 23, Universität Heidelberg 1991, beschrieben. Es können aber auch andere aus dem Stand der Technik bekannte Adhäsionspromotoren, wie auch als Spacer verwendetete Reagenzien, verwendet werden.

Zur Herstellung der erfindungsgemäßen künstlichen Implantate kann das radioaktiv markierte, antithrombogene Polymer aber auch direkt auf die Oberfläche des Substrats aufgebracht werden.

Ferner kann bei Verwendung eines Adhäsionspromotors zuerst der Adhäsionspromotor auf die Oberfläche des Substrats, wie vorstehend ausgeführt, aufgebracht und gegebenenfalls daran gekoppelt werden, und danach wird das radioaktiv markierte, antithrombogene Polymer auf die mit dem Adhäsionspromotor beschichtete Oberfläche des Substrats aufgebracht und gegebenenfalls an den Adhäsionspromotor gekoppelt.

Vorzugsweise wird zur Herstellung der erfindungsgemäßen künstlichen Implantate das antithrombogene Polymer naßchemisch bzw. in Lösung oder aus der Schmelze aufgebracht und gegebenenfalls an den Adhäsionspromotor gekoppelt.

Vor dem Aufbringen des radioaktiv markierten Polydichlorphosphazen, des Adhäsionspromotors oder des radioaktiv markierten, antithrombogenen Polymers kann die Oberfläche des Substrats oxidativ gereinigt werden. Die oxidative Reinigung von Oberflächen mit gleichzeitiger Hydroxylierung, wie sie beispielsweise für Implantate aus Kunststoffen, Metallen oder Keramik eingesetzt werden kann, ist in Ulman Abraham, Analysis of Surface Properies, "An Introduction to Ultrathin Organic Films", 108, 1991, beschrieben.

Zusammengefaßt kann festgestellt werden, daß die erfindungsgemäßen, radioaktiv markierten Implantante, insbesondere Stents, Herzklappen, künstliche Blutgefäße oder sonstige Implantate ohne direkten Blutkontakt, mittels des zuvor beschriebenen Verfahrens vorteilhafterweise einfach herzustellen sind, da keine technisch aufwendige lonenimplantation von radioaktivem Material, beispielsweise ³²P, in das Implantatmaterial nötig ist, sondern das beispielsweise β-Strahlung emittierende Material als Polymerüberzug aufgebracht wird. Dieses Aufbringen kann auch durch die Anwendung der im Bereich der Beschichtung aus dem Stand der Technik bekannten Verfahren, wie z. B. Spin-Coating, Aufrakeln etc. geschehen und betrifft nicht nur das ³²P-Polyphosphazen, sondern auch die Aufbringung des Adhäsionspromotors.

Die erfindungsgemäßen Implantate behalten überraschenderweise die ausgezeichneten mechanischen Eigenschaften des Vorrichtungs- bzw. Implantatmaterials als Substrat bei. Durch den das erfindungsgemäße antithrombogene Polymer enthaltenden Überzug, beispielsweise aufgebracht durch direkte Abscheidung aus der Lösung, weisen die erfindungsgemäßen Implantate nicht nur antithrombogene Eigenschaften auf, was die Biokompatibilität derartiger künstlicher Implantate drastisch verbessert, sondern wird auch unkontrolliertes Zellwachstum, welches beispielsweise nach einer Stentimplantation zu Restenosen führt, infolge der emittierten radioaktiven Strahlung verringert.

Ebenso hat sich gezeigt, daß sich beispielsweise radioaktiv markiertes Poly[bis(trifluoroethoxy)phosphazen] mit und ohne Adhäsionspromotoren naßchemisch oder durch Aufschmelzen direkt immobilisieren läßt. Der Erfolg dieses Präparationsschrittes läßt sich anhand von XPS-Spektroskopie nachweisen.

Sowohl die direkte Beschichtung oder Aufschmelzung mit beispielsweise radioaktiv markiertem Poly[bis(trifluoroethoxy)phosphazen], als auch die Abscheidung von radioaktiv markiertem Polydichlorphosphazen und Veresterung mit beispielsweise 2,2,2-Natriumfluorethanolat kann
- mit oder ohne Trockenschritte im Vakuum, unter Luft oder Schutzgas im Temperaturintervall von beispielsweise etwa -20°C bis etwa 300°C, bevorzugt 0°C bis 200°C und besonders bevorzugt von 20°C bis 100°C, durchgeführt werden, und
- über einen weiten Konzentrationbereich der Ausgangsstoffe und mit unterschiedlichen Zeitintervallen, z.B. aus der Schmelze oder an Lösungen in entsprechenden Lösungsmitteln für Poly[bis(trifluorethoxy)phosphazen], Polydichlorphosphazen und 2,2,2-Natriumtrifluorethanol durchgeführt werden, bevorzugt aus Schmelzen der reinen Stoffe und aus beispielsweise 0,01 molaren Lösungen über einen Zeitraum von 10 Sekunden bis 100 Stunden.

Die vorliegende Erfindung wird nachstehend durch Beispiele näher erläutert.

Zur oxidativen Reinigung und gleichzeitigen Hydroxylierung der künstlichen Implantatoberflächen wird das Substrat in eine Mischung aus 30%iger H₂O₂ und konzentrierter Schwefelsäure (Carosche Säure) im Verhältnis 1:3 für 2 Stunden bei einer Reaktionstemperatur von 80°C gelegt. Nach dieser Behandlung wird das Substrat mit 0,5 L entmineralisiertem Wasser von 18 MOhmcm und etwa pH 5 gewaschen und anschließend im Stickstoffstrom getrocknet. Dieser Reinigungs- und Oxidationsschritt wird in den nachfolgenden erfindungsgemäßen Beispielen, soweit nicht anders erwähnt, als erster Schritt durchgeführt.

Die für das Arbeiten mit radioaktiven Materialien sind aus den Lehrbüchern über radiochemische Arbeitsweisen zu ersehen. Weitere Informationen über notwendige und gesetzlich vorgeschriebene Verhaltensweisen, Schutzmaßnahmen sowie Entsorgungsvorschriften sind der deutschen Verordnung über Strahlenschutz zu entnehmen. Diese Maßnahmen gelten ab dem Moment, ab welchem mit radioaktiven Isotopen gearbeitet wird.

Das dem radioaktiv markierten Poly[bis(trifluoroethoxy)phosphazen] zugrundeliegende ³²P-markierte Polydichlorphosphazen läßt sich gemäß den im Stand der Technik beschriebenen Methoden, ausgehend von der Kondensation von ³²PCl₅, entweder als Reinsubstanz oder mit herkömmlichem, d.h. nicht radioaktiv markiertem, PCl₅ gemischt, mit NH₄Cl, herstellen. Die anschließende Polymerisation des radioaktiv markierten Hexachlorcyclotriphosphazens wird in einer Ampulle mit einem Durchmesser von 5,0 mm bei 250°C ± 1°C sowie einem in der Ampulle herrschenden Druck von 10-2 mm Hg durchgeführt.

### Beispiel 1

Eine 0,1 M Lösung von ³²P-markiertem Polydichlorphosphazen wird unter Inertgasatmosphäre hergestellt (0,174 g auf 5 ml Lösungsmittel). Als Lösungsmittel wird absolutes Toluol verwendet. In diese Lösung wird dann das oxidativ gereinigte künstliche Implantat unter Inertgasatmosphäre bei Raumtemperatur für 24 Stunden eingebracht. Danach wird das so auf dem künstlichen Implantat immobilisierte radioaktiv markierte Polydichlorphosphazen mit 2,2,2-Natriumtrifluoroethanolat in absolutem Tetrahydrofuran als Lösungsmittel (8 ml absolutes Tetrahydrofuran, 0,23 g Natrium, 1,46 ml 2,2,2-Trifluorethanol) verestert. Das Reaktionsgemisch wird während der ganzen Reaktionszeit unter Rückfluß gekocht. Die Veresterung wird unter Inertgasatmosphäre bei 80°C und einer Reaktionszeit von 3 Stunden durchgeführt. Danach wird das so mit dem Überzug beschichtete Substrat mit 4-5 ml absolutem Tetrahydrofuran gewaschen und im Stickstoffstrom getrocknet.

Nach diesen Behandlungen wurde die Oberfläche mit Hilfe der Röntgenphotoelektronenspektroskopie auf die elementare Zusammensetzung, Stöchiometrie und Überzugsdicke untersucht. Die Ergebnisse zeigen, daß alle Reaktionsschritte erfolgt sind und Überzugsdicken von größer als 3,4 nm erreicht wurden.

### Beispiel 2

Das mit Caroscher Säure oxidativ gereinigte künstliche Implantat wird 30 Minuten in eine 2%-ige Aminopropyltrimethoxysilanlösung in absolutem Ethanol eingetaucht. Daraufhin wird das Substrat mit 4-5 ml absolutem Ethanol gewaschen und 1 Stunde im Trockenschrank bei 105°C belassen.

Nach der Ankopplung des Aminopropyltrimethoxysilans an die oxidativ gereinigte Oberfläche des Substrats wird das so behandelte Substrat für 24 Stunden bei Raumtemperatur unter Inertgasatmosphäre in eine 0,1 M Lösung von radioaktiv markiertem Polydichlorphosphazen in absolutem Toluol eingebracht. Anschließend wird das so behandelte künstliche Implantat unter Inertgasatmosphäre mit 4-5 ml absolutem Toluol gewaschen, danach in eine frisch hergestellte 2,2,2-Natriumtrifluorethanolatlösung (8 ml absolutes Tetrahydrofuran, 0,23 g Natrium, 1,46 ml 2,2,2-Trifluorethanol) eingebracht und 3 Stunden bei 80°C unter Rückfluß und Inertgasatmosphäre gekocht. Zuletzt wird das so hergestellte künstliche Implantat mit 4-5 ml absolutem Tetrahydrofuran gewaschen und im Stickstoffstrom getrocknet.

Die Oberfläche wurde nach dieser Behandlung mit Hilfe der Photoelektronenspektroskopie auf die elementare Zusammensetzung, Stöchiometrie und Überzugsdicke untersucht. Die Ergebnisse zeigen, daß die jeweiligen Ankopplungen erfolgten und Überzugsdicken von größer als 5,5 nm erreicht wurden.

### Beispiel 3

Das mit Caroscher Säure oxidativ gereinigte künstliche Implantat wird in eine 2%-ige Aminopropyltrimethoxysilanlösung in absolutem Ethanol für 30 Minuten bei Raumtemperatur eingetaucht. Daraufhin wird das Substrat mit 4-5 ml absolutem Ethanol gewaschen und eine Stunde bei 105°C im Trockenschrank belassen. Nach der Ankopplung des Aminopropyltrimethoxysilans an die Oberfläche des Substrats wird das so behandelte künstliche Implantat 24 Stunden bei Raumtemperatur in eine 0,1 M Lösung von radioaktiv markiertem Poly[bis(trifluorethoxy)phosphazen] in Ethylacetat (0,121 g auf 5 ml Ethylacetat) eingebracht. Anschließend wird das so hergestellte künstliche Implantat mit 4-5 ml Ethylacetat gewaschen und im Stickstoffstrom getrocknet.

Nach dieser Behandlung wurde die Oberfläche mittels der Photoelektronen-spektroskopie auf die elementare Zusammensetzung, Stöchiometrie und Überzugsdicke untersucht. Die Ergebnisse zeigen, daß die Immobilisierung des radioaktiv markierten Poly[bis(trifluoroethoxy)phosphazens] über das Aminopropyltrimethoxysilan als Adhäsionspromotor erfolgt ist und Überzugsdicken von über 2,4 nm erreicht wurden.

### Beispiel 4

Das mit Caroscher Säure oxidativ gereinigte künstliche Implantat wird bei 70°C in eine 0,1 M Lösung von radioaktiv markierten Poly[bis(trifluoroethoxy)-phospazen] in Ethylacetat (0,121 g auf 5 ml Ethylacetat) für 24 Stunden gelegt. Anschließend wird da so behandelte künstliche Implantat mit 4-5 ml Ethylacetat gewaschen und im Stickstoffstrom getrocknet.

Das so hergestellte künstliche Implantat wurde mit Hilfe der Photoelektronenspektroskopie auf die elementare Zusammensetzung, Stöchiometrie und Überzugsdicke untersucht. Die Ergebnisse zeigen, daß die Ankopplung des radioaktiv markierten Poly[bis(trifluoroethoxy)phosphazens] auf der Implantatoberfläche erfolgt ist und Schichtdicken von über 2,1 nm erreicht wurden.

### Beispiel 5

Das mit Caroscher Säure oxidativ gereinigte künstliche Implantat wird bei 70°C in die Schmelze des radioaktiv markierten Poly[bis(trifluoroethoxy)phosphazens] gelegt und dort für etwa 10 Sekunden bis etwa 10 Stunden belassen. Anschließend wird das so behandelte Implantat mit 4-5 ml Ethylacetat gewaschen und im Stickstoffstrom getrocknet.

Das so hergestellte künstliche Implantat wurde mit Hilfe der Photoelektronenspektroskopie auf die elementare Zusammensetzung, Stöchiometrie und Überzugsdicke untersucht. Die Ergebnisse zeigen, daß die Ankopplung des radioaktiv markierten Poly[bis(trifluoroethoxy)phosphazens] auf der Implantatoberfläche erfolgt ist und beliebige Schichtdicken bis zu einigen Millimetern erreicht wurden.

## Patentansprüche

1. Antithrombogenes Polymer mit der folgenden allgemeinen Formel (I), 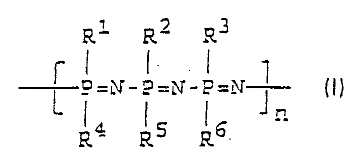 wobei n für 2 bis ∞ steht, R¹ bis R⁶ gleich oder unterschiedlich sind und einen Alkoxy-, Alkylsulfonyl-, Dialkylamino- oder Aryloxyrest oder einen Heterocycloalkyl- oder Heteroarylrest mit Stickstoff als Heteroatom bedeuten und wobei mindestens ein Teil der Polymerkette des antithrombogenen Polymers einen radioaktiv markierten Bestandteil enthält.

2. Antithrombogenes Polymer nach Anspruch 1, wobei der radioaktiv markierte Bestandteil beim radioakiven Zerfall β-Strahlung oder γ-Strahlung emittiert.

3. Antithrombogenes Polymer nach Anspruch 1 oder 2, welches ein radioaktives Isotop der V. Hauptgruppe enthält.

4. Antithrombogenes Polymer nach Anspruch 3, welches ein radioaktives Phosphorisotop enthält.

5. Antithrombogenes Polymer nach Anspruch 4, wobei das Phosphorisotop ³²P ist.

6. Antithrombogenes Polymer nach Anspruch 5, wobei das ³²P-Phosphorisotop in der Polymerkette des antithrombogenen Polymers statistisch verteilt ist.

7. Antithrombogenes Polymer nach Anspruch 5, wobei jedes Phosphoratom in der Polymerkette des antithrombogenen Polymers ein ³²P-Isotop ist.

8. Antithrombogenes Polymer nach einem der Ansprüche 1 bis 7, wobei mindestens einer der Reste R¹ bis R⁶ ein Alkoxyrest ist, der mit mindestens einem Fluoratom substituiert ist.

9. Antithrombogenes Polymer nach einem der Ansprüche 1 bis 8, welches ³²P-markiertes Poly[bis(trifluoroethoxy)phosphazen] ist.

10. Antithrombogenes Polymer nach einem der Ansprüche 1 bis 9 zur Verwendung als Bestandteil therapeutischer Vorrichtungen zur Verhinderung von übermäßiger Zellproliferation oder Narbenbildung oder zur Turnorbehandlung.

11. Antithrombogenes Polymer nach Anspruch 10, wobei die Vorrichtung aus künstlichen Implantanten, Pflastern, Herzklappen, künstlichen Blutgefäßen, Stents, Kathetern, Urethern oder sonstigen Implantaten ohne direkten Blutkontakt ausgewählt ist.

12. Therapeutische Vorrichtung, umfassend ein antithrombogenes Polymer nach einem der Ansprüche 1 bis 9.

13. Vorrichtung nach Anspruch 12, welche ein Pflaster ist.

14. Vorrichtung nach Anspruch 12, welche ein künstliches Implantat ist.

15. Vorrichtung nach Anspruch 14, wobei das künstliche Implantat ein Implantatmaterial als Substrat und einen mindestens teilweise auf die Oberfläche des Substrats aufgebrachten, biokompatiblen Überzug, der das vorgenannt definierte antithrombogenes Polymer enthält, umfaßt.

16. Vorrichtung nach Anspruch 15, wobei zwischen der Oberfläche des Substrats und dem biokompatiblen Überzug eine Schicht angeordnet ist, die einen Adhäsionspromotor enthält.

17. Vorrichtung nach Anspruch 16, wobei der Adhäsionspromotor eine Silizium-organische Verbindung ist.

18. Vorrichtung nach Anspruch 17, wobei die Silizium-organische Verbindung Aminopropyltrimethoxysilan ist.

## Claims

1. Antithrombogenic polymer having the following general formula (I) 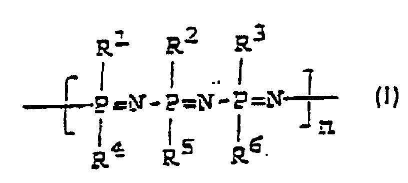 where n is from 2 to 4, R¹ to R⁶ are identical or different and are an alkoxy, alkylsulphonyl, dialkylamino or aryloxy radical or a heterocycloalkyl or heteroaryl radical with nitrogen as heteroatom, and where at least part of the polymer chain of the antithrombogenic polymer contains a radioactively labelled constituent.

2. Antithrombogenic polymer according to Claim 1, where the radioactively labelled constituent emits β rays or γ rays on radioactive decay.

3. Antithrombogenic polymer according to Claim 1 or 2, which contains a radioactive isotope of main group V.

4. Antithrombogenic polymer according to Claim 3, which contains a radioactive phosphorus isotope.

5. Antithrombogenic polymer according to Claim 4, where the phosphorus isotope is ³²P.

6. Antithrombogenic polymer according to Claim 5, where the ³²P phosphorus isotope is randomly distributed in the polymer chain of the antithrombogenic polymer.

7. Antithrombogenic polymer according to Claim 5, where each phosphorus atom in the polymer chain of the antithrombogenic polymer is a ³²P isotope.

8. Antithrombogenic polymer according to any of Claims 1 to 7, where at least one of the radicals R¹ to R⁶ is an alkoxy radical which is substituted by at least one fluorine atom.

9. Antithrombogenic polymer according to any of Claims 1 to 8, which is ³²P-labelled poly[bis(trifluoroethoxy)phosphazene].

10. Antithrombogenic polymer according to any of Claims 1 to 9 for use as constituent of therapeutic devices for preventing excessive cell proliferation or scar formation or for treating tumours.

11. Antithrombogenic polymer according to Claim 10, where the device is selected from artificial implants, plasters, heart valves, artificial blood vessels, stents, catheters, urethers or other implants without direct contact with blood.

12. Therapeutic device comprising an antithrombogenic polymer according to any of Claims 1 to 9.

13. Device according to Claim 12, which is a plaster.

14. Device according to Claim 12, which is an artificial implant.

15. Device according to Claim 14, where the artificial implant comprises an implant material as substrate and a biocompatible coating which is applied to at least part of the surface of the substrate and which contains the antithrombogenic polymer defined as mentioned above.

16. Device according to Claim 15, where a layer which contains an adhesion promoter is disposed between the surface of the substrate and the biocompatible coating.

17. Device according to Claim 16, where the adhesion promoter is an organosilicon compound.

18. Device according to Claim 17, where the organosilicon compound is aminopropyltrimethoxysilane.

## Revendications

1. Polymère antithrombogène répondant à la formule générale (I) suivante 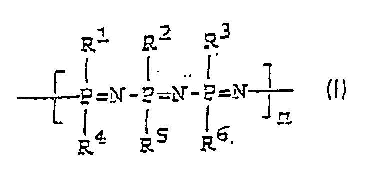 dans laquelle n a une valeur de 2 à l'infini, R¹ à R⁶ sont identiques ou différents et représentent un reste alkoxy, alkyl-sulfonyle, dialkylamino ou aryloxy, ou bien un reste hétérocycloalkyle ou hétéroaryle contenant de l'azote comme hétéroatome et une partie au moins de la chaîne du polymère antithrombogène contient un constituant marqué par la radioactivité.

2. Polymère antithrombogène suivant la revendication 1, dans lequel le constituant marqué par la radioactivité émet un rayonnement β ou un rayonnement γ au cours de la désintégration radioactive.

3. Polymère antithrombogène suivant la revendication 1 ou 2, qui contient un isotope radioactif du groupe principal V.

4. Polymère antithrombogène suivant la revendication 3, qui contient un isotope radioactif du phosphore.

5. Polymère antithrombogène suivant la revendication 4, dans lequel l'isotope du phosphore est ³²P.

6. Polymère antithrombogène suivant la revendication 5, dans lequel l'isotope de phosphore ³²P est réparti statistiquement dans la chaîne du polymère antithrombogène.

7. Polymère antithrombogène suivant la revendication 5, dans lequel chaque atome de phosphore présent dans la chaîne du polymère antithrombogène est un isotope ³²P.

8. Polymère antithrombogène suivant l'une des revendications 1 à 7, dans lequel l'un au moins des restes R¹ à R⁶ est un reste alkoxy qui est substitué avec au moins un atome de fluor.

9. Polymère antithrombogène suivant l'une des revendications 1 à 8, qui est un poly[bis(trifluoréthoxy)-phosphazène] marqué au ³²P.

10. Polymère antithrombogène suivant l'une des revendications 1 à 9, destiné à être utilisé comme composant de dispositifs thérapeutiques pour inhiber la prolifération excessive de cellules ou la formation de cicatrices ou pour le traitement de tumeurs.

11. Polymère antithrombogène suivant la revendication 10, pour lequel le dispositif est choisi entre des implants artificiels, des emplâtres, des valvules cardiaques, des vaisseaux sanguins artificiels, des extenseurs, des cathéters, des urètres ou d'autres implants sans contact direct avec le sang.

12. Dispositif thérapeutique comprenant un polymère antithrombogène suivant l'une des revendications 1 à 9.

13. Dispositif suivant la revendication 12, qui est un emplâtre.

14. Dispositif suivant la revendication 12, qui est un implant artificiel.

15. Dispositif suivant la revendication 14, dans lequel l'implant artificiel comprend un matériau pour implant comme substrat et un revêtement biocompatible, appliqué au moins partiellement sur la surface du substrat, qui contient le polymère antithrombogène défini ci-dessus.

16. Dispositif suivant la revendication 15, dans lequel une couche qui contient un activateur d'adhérence est disposée entre la surface du substrat et le revêtement biocompatible.

17. Dispositif suivant la revendication 16, dans lequel le promoteur d'adhérence est un composé organique de silicium.

18. Dispositif suivant la revendication 17, dans lequel le composé organique de silicium est l'aminopropyltriméthoxysilane.
